# EUROPÄISCHE PATENTSCHRIFT

(11) **EP 1 360 331 B1**
(45) Veröffentlichungstag und Bekanntmachung des Hinweises auf die Patenterteilung: **12.12.2007**
(21) Anmeldenummer: 02712733.1
(22) Anmeldetag: 01.02.2002
(51) Int. Cl.: C12Q 1/68

(54) **VEKTORSYSTEM ZUM SCREENING VON WIRKSTOFFEN**
VECTOR SYSTEM USED IN SCREENING ACTIVE SUBSTANCES
SYSTEME VECTEUR POUR LE CRIBLAGE DE PRINCIPES ACTIFS

(30) Priorität: 03.02.2001 DE 10104952
(43) Veröffentlichungstag der Anmeldung: 12.11.2003
(73) Patentinhaber: MAX-DELBRÜCK-CENTRUM FÜR MOLEKULARE MEDIZIN, 13125 Berlin (DE)
(72) Erfinder: CALKHOVEN, Cornelis, F., 16341 Zepernick (DE); LEUTZ, Achim, 16352 Schönwalde (DE)
(74) Vertreter: Hertin, Paul W.
(86) Internationale Anmeldenummer: PCT/DE2002/000367
(87) Internationale Veröffentlichungsnummer: WO 2002/063036

(56) Entgegenhaltungen:
- EP-A- 1 176 196
- WO-A-97/24460
- US-A1- 2002 006 605
- CALKHOVEN COR F ET AL: "Translational control of C/EBPalpha and C/EBPbeta isoform expression." GENES & DEVELOPMENT, Bd. 14, Nr. 15, 1. August 2000 (2000-08-01), Seiten 1920-1932, XP002204522 ISSN: 0890-9369

## Beschreibung

Die Erfindung betrifft ein Vektorsystem zum Screening von Wirkstoffen, welche die Translation in der Zelle beeinflussen. Anwendungsgebiete der Erfindung sind die pharmazeutische Industrie, die Medizin und die Molekularbiologie.

Die Kontrolle der Boten RNA Translation in Proteine ist ein wichtiger biologischregulatorischer Vorgang, dessen Deregulation Ursache vieler Krankheiten ist. Es gibt bereits einige Arbeiten, die sich mit der Pathologie der Translationsregulation von Proteinen in der Zelle beschäftigen.

Beispielsweise wurde eine erhöhte Expression des eukaryontischen Translations-Initiationsfaktors eIF-4E beim Darmkrebs gefunden. (Rosenwald et al., 1999; Oncogene 18:2507). Dieser Initiationsfaktor bindet die 5' gelegene CAP-Struktur eukaryontischer mRNAs und spielt somit bei der Initiation der Translation eine entscheidende Rolle. Zuvor war bereits gezeigt worden, dass die Überexpression von eIF-4E normale Nagerzellen in Tumorzellen umwandelt und zu einer Verstärkung von Transformationsparametern in menschlichen Zellen führt. Andererseits unterdrückt die Ausschaltung von eIF-4E die Transformation durch das ras Onkogen. Dies weist auf eine grundsätzliche Rolle der Translationsregulation bei der Entstehung von Krebs hin. In diesem Zusammenhang wurde kürzlich festgestellt, dass die mTOR Kinase, der eine grundlegende Funktion in der Translationsregulation zukommt, auch eine Schlüsselrolle bei der Transformation durch die Oncogene P3K und Akt spielt (Aoki et al., 2001; PNAS, 98:136). Neben der Tumorigenese spielt die Regulation der Translation auch bei metabolischen Syndromen eine Rolle. Beim autosomal rezessiven "Wolcott-Rallison" Syndrom z. B. wurden als Ursache Mutationen im EIF2AK3 Gen gefunden (Delepine et al., 2000; Nat. Gen. 25:406). Das EIF2AK3 Gen kodiert für eine Kinase, die den Translations-Initiationsfaktor elF2alpha phosphoryliert. Eine Reihe weiterer Funktionen der translationalen Kontrolle im Metabolismus, bei der Embryonalentwicklung, der Zelldifferenzierung und der Infektabwehr sind inzwischen recht gut bekannt und in dem kürzlich erschienenen Band "Translational Control of Gene Expression", (ed. by Sonenberg, Hershey and Mathews, Cold Spring Harbor Laboratory Press, CSH, N.Y., 2000; ISSN 0270-1847; 39) zusammengefasst.

Der Erfindung liegt die Aufgabe zugrunde, ein Vektorsystem aufzubauen, welches eine unmittelbare Beobachtung von Translationsvorgängen in der Zelle erlaubt.

Die Erfindung wird gemäß dem Hauptanspruch realisiert, die Unteransprüche sind Vorzugsvarianten.

Kernstück der Erfindung ist ein neues rekombinantes Reporterplasmidkonstrukt (Vektorsystem), welches im folgenden näher charaktrisiert wird. Es besteht aus einer Reihe von Modulen. Diese Module dienen i) als Kontrollelemente, welche die Initiation der Translation oder die Funktion von Ribosomen beinflussen, ii) zur Feststellung von Regulationsvorgängen und zum internen Abgleich (z. B. Transkription vs. Translation), iii) zur vereinfachten Messung mit hohem Probendurchsatz (Sezernierung der Translationsprodukte aus Zellen und Immobilisierung im Microformat, Reinigung und Detektion mit Hilfe von antigenen "Tags").
1. Das Konstrukt enthält Kontrollelemente der Translation. Diese Elemente liegen im wesentlichen 5' von der Detektionskassette (s. u.), können aber auch 3' oder innerhalb der Detektionskassette liegen. Die Kontrollelemente steuern die differentielle Expression unterschiedlicher Protein-Reporterkonstrukte, die in der Detektionskassette kodiert sind.
2. Das Konstrukt enthält eine Detektionskassette. Diese Detektionskassette kodiert für zwei oder mehrere Protein-Reporterkonstrukte in gleichen oder in unterschiedlichen Leserastern. Differentielle Initiation der Translation führt zur Verschiebung der Reportermengen, die somit differentielle Translation widerspiegeln. Regulationsvorgänge, die zu einer allgemeinen Veränderung der Translation führen, bewirken hingegen eine gleichartige Verschiebung der Reporterexpression. Beide Typen der Regulation können gegeneinander verrechnet und in mathematischen Modellen zusammengefasst werden. Dies ermöglicht eine computergesteuerte Auswertung der Ergebnisse und eine Automatisierung des Verfahrens.
3. Die Reporterkonstrukte enthalten Signalsequenzen zum Ausschleusen aus der Zelle. Somit können Reporterproteine wahlweise in Zelllysaten oder im Überstand bestimmt werden. Die letztere Eigenschaft dient insbesondere zur High Throughput Analyse potentieller Wirkstoffe.
4. Die Reporterkonstrukte enthalten verschiedene antigene Stellen, mit deren Hilfe Reporterproteine immobilisiert und differentiell detektiert werden können. Eine antigene Stelle dient zur Immobilisierung der Reporterproteine, eine weitere, und in den jeweiligen Reporterproteinen verschiede antigene Stelle dient zur differentellen Reporter-Detektion (siehe Beispiel 1).

Mit Hilfe dieses modular aufgebauten rekombinanten Reporterplasmidkonstrukts Translation Control Reporter System (TCRS) werden Substanzen auf ihre Wirkung hin untersucht, in Vorgänge der differentiellen Initiation der Translation sowie in vielfältige Vorgänge bei der Translation einzugreifen. Das TCRS Konstrukt wird in Zellen transient oder stabil eingebracht. Sodann werden die Zellen mit Substanzen beschickt und die resultierenden, von dem Konstrukt stammenden Reportermengen bestimmt und miteinander verglichen. Aus den ermittelten Daten werden translationelle Kontrollvorgänge eruiert.

Mit Hilfe des erfindungsgemäßen Systems wird die Grundlage für das schnelle Auffinden und Optimieren von Wirksubstanzen geschaffen, die translationelle Kontrollvorgänge beeinflussen und zur pharmakologischen Korrektur von Krankheiten dienen können. Darüberhinaus eignet sich das System zur wissenschaftlichen Analyse translationeller Regulationsvorgänge.

Die Erfindung soll nachfolgend durch Ausführungsbeispiele näher erläutert werden.

### Beispiel 1

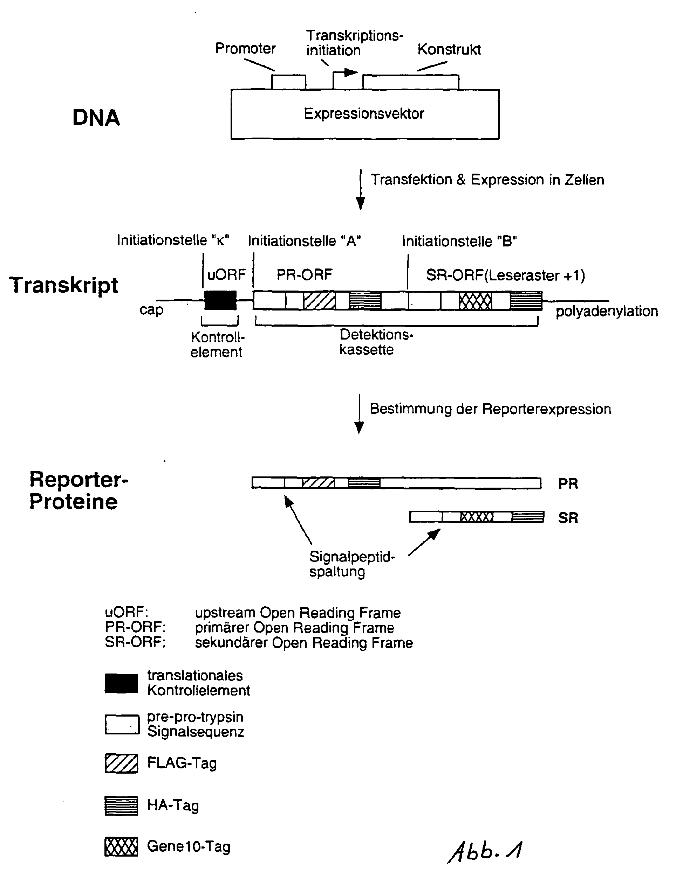

Ein Prototyp des erfindungsgemäßen Konstrukts mit folgenden Eigenschaften liegt vor (vergleiche Abb. 1):

Der eukaryontische Expressionsvektor ist folgendermassen aufgebaut: ca 50 Basenpaare der Anfangsequenz ("Leadersequence") aus der beta-globin mRNA. Danach folgt 3' der upstream Open Reading Frame (uORF) des Ratten C/EBP alpha Gens als translationelles Kontrollelement mit der Initiationsstelle "K". Danach folgt die Detektionskassette mit den beiden Reportergenen PR und SR. "A" bezeichnet die Initiationsstelle des PR Leserasters. PR enthält eine Signalsequenz (pre-pro-Trypsin), die zur Ausschleussung des primären Reporterproteins (PR) führt. Als antigenes Epitop schliesst sich zuerst eine FLAG Sequenz und dann ein Hämagglutinin (HA) Epitop an. Letzteres dient zur Immobilisierung des Reporterproteins. Von der Initiationsstelle (B) wird SR kodiert. SR ist gegenüber PR um ein Nukleotid nach 3' verschoben. Beim Durchlesen der an "A" gestarteten Ribosomen ergibt sich wegen der Leserasterverschiebung kein sinnvolles SR Produkt. Durch Initiation an B hingegen entsteht ein von PR verschiedenes, sekundäres Reporterprotein (SR). SR besitzt ebenso wir PR eine pre-pro-Trypsin Signalsequenz am N-Terminus. Es schliesst sich ein Gene10 Antigenepitop sowie ein HA-Epitop an. Beide Reporterkonstrukte werden 3' überlappend terminiert. Reporterproteinexpression von der A oder B Initiationsstelle wird durch das Kontrollelement (uORF, κ) beinflusst.

### Beispiel 2

Das Konstrukt (Abb. 1) wurde in Zellen transfiziert und nach 24 und 48 Stunden wurde das Reporterprotein bestimmt. Es zeigte sich, dass Reporterproteine mit den gewünschten Eigenschaften (Grösse, Sezernierung, antigene Eigenschaften) produziert werden. Ein ähnliches Konstrukt, jedoch ohne das translationelle Kontrollelement, produziert im Vergleich mit TCRS andere Mengen eines Reporterproteins. Somit eignet sich das vorliegende TCRS Konstrukt zum High Throughput Screenining von Substanzen, welche die Translation durch das Kontrollelement beeinflussen.

## Patentansprüche

1. Vektorsystem zum Screening von Wirkstoffen, bestehend aus
- Kontrollelementen der Translation, die im wesentlichen 5' von der Detektionskassette liegen, ggf. aber auch in 3'-Stellung oder innerhalb der Detektionskassette,
- einer Detektionskassette, die für zwei oder mehrere Protein-Reporterkonstrukte in unterschiedlichen Leserastern kodiert,
- wobei die Reporterkonstrukte
- Signalsequenzen zum Ausschleusen aus der Zelle und
- verschiedene antigene Stellen, mit deren Hilfe Reporterproteine immobilisiert und differentiell detektiert werden können, enthalten.

2. Vektorsystem zum Screening von Wirkstoffen nach Anspruch 1, **dadurch gekennzeichnet, dass** es auf abgeleiteten Kontrollelementen mit alternativen Reportergenen wie z. B. GFP, Luciferase zum modifizierten Nachweis gekoppelt ist.

3. Vektorsystem zum Screening von Wirkstoffen gemäß Abbildung 1 .

4. Vektorsystem zum Screening von Wirkstoffen, **dadurch gekennzeichnet, dass** das Vektorsystem nach einem der Ansprüche 1 bis 3 in Zellen transient oder stabil eingebracht wird, sodann die Zellen mit Substanzen beschickt werden und die resultierenden, von dem Vektorsystem stammenden Reportermengen bestimmt und miteinander verglichen werden.

5. Zelllinien zum Screening von Wirkstoffen, enthaltend ein Vektorsystem gemäß Anspruch 1 - 3.

6. Verwendung des Vektorsystems nach einem der Ansprüche 1 bis 3 zur Untersuchung der Wirkung von Substanzen auf eine Translation.

7. Verwendung nach dem vorhergehenden Anspruch,
**dadurch gekennzeichnet, dass**
Vorgänge einer differentiellen Initiation der Translation untersucht werden.

8. Verwendung nach einem der vorhergehenden Ansprüche zum High Throughput Screening von Substanzen, die die Translation durch ein Kontrollelement beeinflussen.

9. Verwendung nach einem der vorhergehenden Ansprüche zur Analyse translationeller Regulationsvorgänge.

10. Verwendung nach einem der vorhergehenden Ansprüche zur Detektion von Substanzen, die translationelle Kontrollvorgänge beeinflussen.

## Claims

1. A vector system for screening active substances, consisting of
- control elements of translation situated essentially 5' from the detection cassette, but optionally also in 3' position or within the detection cassette,
- a detection cassette encoding two or more protein reporter constructs in different reading frames,
said reporter constructs containing
- signal sequences for release from the cell, and
- various antigenic sites by means of which reporter proteins can be immobilized, and detected differentially.

2. The vector system for screening active substances according to claim 1, **characterized in that** it is coupled with alternative reporter genes, such as GFP, luciferase, on derived control elements for modified detection.

3. A vector system for screening active substances in accordance with Figure 1.

4. A vector system for screening active substances, **characterized in that** the vector system according to any of claims 1 to 3 is transiently or stably incorporated in cells, the cells are subsequently supplied with substances, and the resulting reporter quantities coming from the vector system are determined and compared with each other.

5. Cell lines for screening active substances, including a vector system according to claims 1 to 3.

6. Use of the vector system according to any of claims 1 to 3 for investigating the effect of substances on translation.

7. The use according to the preceding claim,
**characterized in that**
processes of differential initiation of translation are investigated.

8. The use according to any of the preceding claims for the high-throughput screening of substances influencing translation via a control element.

9. The use according to any of the preceding claims for the analysis of translational regulatory processes.

10. The use according to any of the preceding claims for the detection of substances influencing processes of translational control.

## Revendications

1. Système vecteur pour le criblage de principes actifs, comprenant
- des éléments de contrôle de translation, lesquels sont situés sensiblement à 5' de la cassette de détection, le cas échéant également dans une position à 3', ou à l'intérieur de la cassette de détection,
- une cassette de détection qui encode pour deux ou plusieurs constructions rapporteuses de protéine dans différentes trames de lecture,
- les constructions rapporteuses contenant
-- des séquences de signaux pour la libération hors de la cellule et
-- différents sites antigènes, à l'aide desquels les protéines rapporteuses peuvent être immobilisées et détectées de façon différentielle.

2. Système vecteur pour le criblage de principes actifs selon la revendication 1, **caractérisé en ce qu'**il est couplé, sur des éléments de contrôle dérivés, à des gènes rapporteurs en variante ou alternatifs tels que GFP, luciférase en vue de l'identification modifiée.

3. Système vecteur pour le criblage de principes actifs selon la figure 1.

4. Système vecteur pour le criblage de principes actifs, **caractérisé en ce que** le système vecteur selon l'une quelconque des revendications 1 à 3 est introduit de façon transitoire ou permanente (stable) dans les cellules, puis **en ce que** les cellules sont alimentées en ou dotées de substances, et **en ce que** les quantités rapporteuses résultantes, provenant du système vecteur sont déterminées et comparées entre elles.

5. Lignées cellulaires pour le criblage de principes actifs, comprenant un système vecteur selon la revendication 1 à 3.

6. Utilisation du système vecteur selon l'une quelconque des revendications 1 à 3 pour étudier l'effet des substances sur une translation.

7. Utilisation selon la revendication précédente,
**caractérisée en ce que**
l'on analyse les processus d'une initiation différentielle de la translation.

8. Utilisation selon l'une quelconque des revendications précédentes pour le criblage à haut débit de substances qui influencent la translation par un élément de contrôle.

9. Utilisation selon l'une quelconque des revendications précédentes pour l'analyse de processus translationnels de régulation.

10. Utilisation selon l'une quelconque des revendications précédentes pour la détection de substances qui influencent les processus translationnels de contrôle.
